Europäisches Patentamt

European Patent Office

Office européen des brevets

(11) Veröffentlichungsnummer: **0 448 122 A2**

## (12) EUROPÄISCHE PATENTANMELDUNG

(21) Anmeldenummer: **91104551.6**

(22) Anmeldetag: **22.03.91**

(51) Int. Cl.5: **A61L 2/18**

(30) Priorität: **23.03.90 DE 4009454**

(43) Veröffentlichungstag der Anmeldung:
**25.09.91 Patentblatt 91/39**

(84) Benannte Vertragsstaaten:
**CH GB IT LI NL**

(71) Anmelder: **Biermaier, Stephan**
**Ulrichstrasse 47**
**W-8904 Friedberg 3(DE)**

(72) Erfinder: **Biermaier, Stephan**
**Ulrichstrasse 47**
**W-8904 Friedberg 3(DE)**

(74) Vertreter: **von Bülow, Tam Dr. et al**
**Samson, Bülow & Partner**
**Widenmayerstrasse 5**
**W-8000 München 22(DE)**

(54) **Desinfektionseinrichtung für Türgriffe, insbesondere für Türgriffe an Desinfektionsspülmaschinen.**

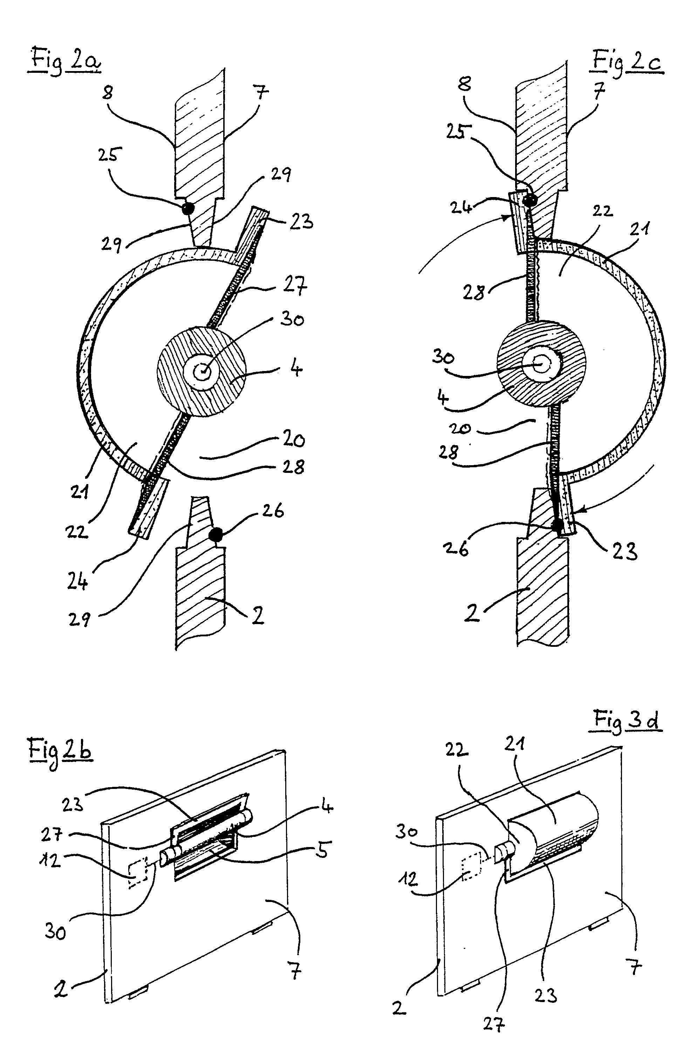

(57) Die Desinfektionseinrichtung für Türgriffe (4) stellt in Abhängigkeit von einer übergeordneten Funktion, wie z.B. Beendigung des Desinfektionsvorganges bei einer Desinfektionsspülmaschine, vorgegebene Zeitintervalle oder vorgegebene Anzahl von Betätigung des Türgriffes (4), zwangsweise eine automatische Desinfektion des Türgriffes sicher. Beispielsweise wird bei einer Desinfektionsspülmaschine der Türgriff dem Wasch- und Desinfektionsvorgang im Inneren der Spülmaschine ausgesetzt (Fig. 2a).

Fig 2a

Die Erfindung bezieht sich auf eine Desinfektionseinrichtung für Türgriffe, insbesondere für Türgriffe an Desinfektionsspülmaschinen gemäß dem Oberbegriff des Patentanspruches 1.

In Kliniken, Labors, Arztpraxen etc. hat sich herausgestellt, daß Türgriffe nur recht unregelmäßig gereinigt werden und daher eine Quelle für Verunreinigungen, Keime, Bakterien usw. darstellen. Insbesondere hat sich herausgestellt, daß beim Beschicken von Desinfektionsspülmaschinen, Sterilisationsapparaten und ähnlichem der Griff des Gerätes kontaminiert wird und daß bei der Entnahme der desinfizierten oder sterilisierten Gegenstände Keime vom Türgriff über der Hand der Bedienperson auf die desinfizierten Gegenstände übertragen werden. Um diese Probleme zu vermeiden mußte bisher der Griff vor jeder Entnahme manuell dekontaminiert werden, was erfahrungsgemäß jedoch oft unterblieb. Zur Lösung dieses Problemes ist es auch bekannt, die Tür ohne Griff auszuführen und durch fußbetätigte Hebel zu öffnen und zu schließen. Diese Konstruktionen sind aber aufwendig und verhindern nicht, daß die Bedienperson reflexartig die meist kontaminierte Außenseite der Tür anfaßt.

Aufgabe der Erfindung ist es, eine Desinfektionseinrichtung zu schaffen, die sicherstellt, daß der Türgriff im Bedarfsfall vor seiner Benutzung desinfiziert ist.

Diese Aufgabe wird durch die im kennzeichnenden Teil des Patentanspruches 1 angegebenen Merkmale gelöst. Vorteilhafte Ausgestaltungen und Weiterbildungen der Erfindung sind den Unteransprüchen zu entnehmen.

Mit der Erfindung wird sichergestellt, daß der Türgriff unabhängig von Eingriffen des Benutzers automatisch desinfiziert wird.

Nach einer ersten Variante der Erfindung wird bei Desinfektionsspülmaschinen der Türgriff während des Spülvorganges in den Spülraum verfahren und dort zusammen mit dem zu reinigenden Gut gereinigt und desinfiziert. Verschiedene Untervarianten dieses Prinzips sehen vor, das Türblatt zu wenden, den Griff selbst in den Waschraum zu verfahren oder den Waschraum durch bewegbare Teile so zu vergrößern, daß der Türgriff in den Waschraum mit einbezogen wird.

Eine andere Variante der Erfindung sieht vor, den Griff benutzerunabhängig mit Desinfektionslösung zu besprühen oder zu tränken.

Eine dritte Grundvariante der Erfindung sieht vor, den Griff mit einer sich selbsttätig erneuernden Schutzfolie zu überziehen.

Eine weitere Grundvariante der Erfindung sieht vor, den Griff thermisch zu desinfizieren.

Bei all diesen Grundvarianten und weiteren, in der nachfolgenden Beschreibung erläuterten Untervarianten ist sichergestellt, daß unabhängig von Nachlässigkeiten der Benutzer der Griff ausreichend desinfiziert ist.

Im folgenden wird die Erfindung anhand von Ausführungsbeispielen im Zusammenhang mit den Zeichnungen ausführlicher erläutert. Es zeigt:

Fig. 1a
ein erstes Ausführungsbeispiel einer Desinfektionseinrichtung nach der Erfindung in Draufsicht auf eine Desinfektionsspülmaschine;

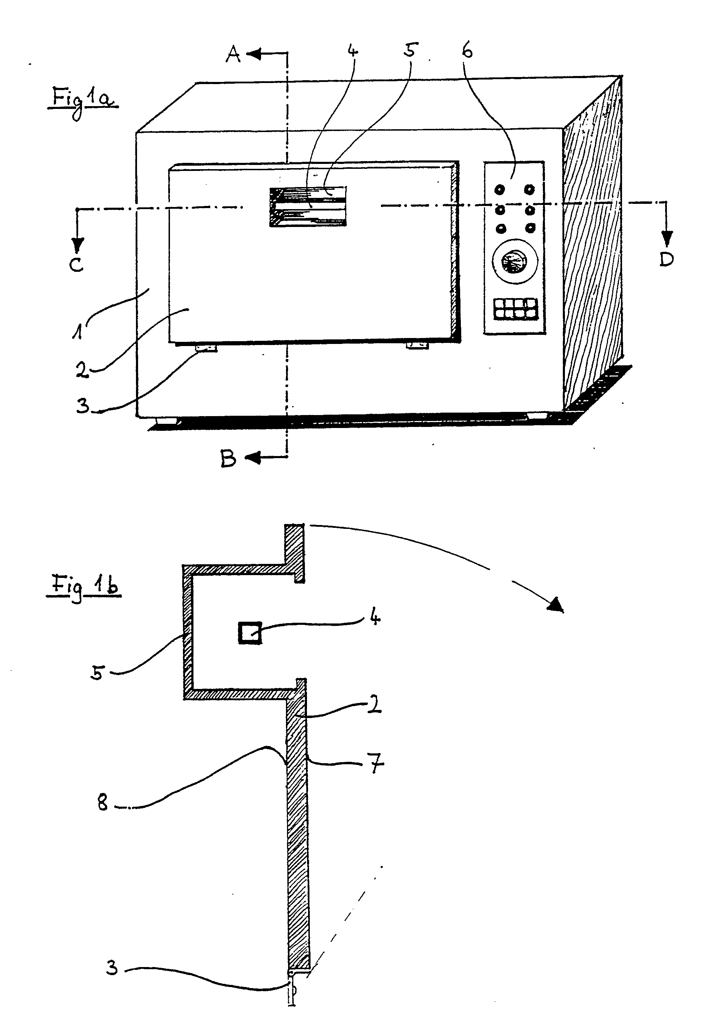

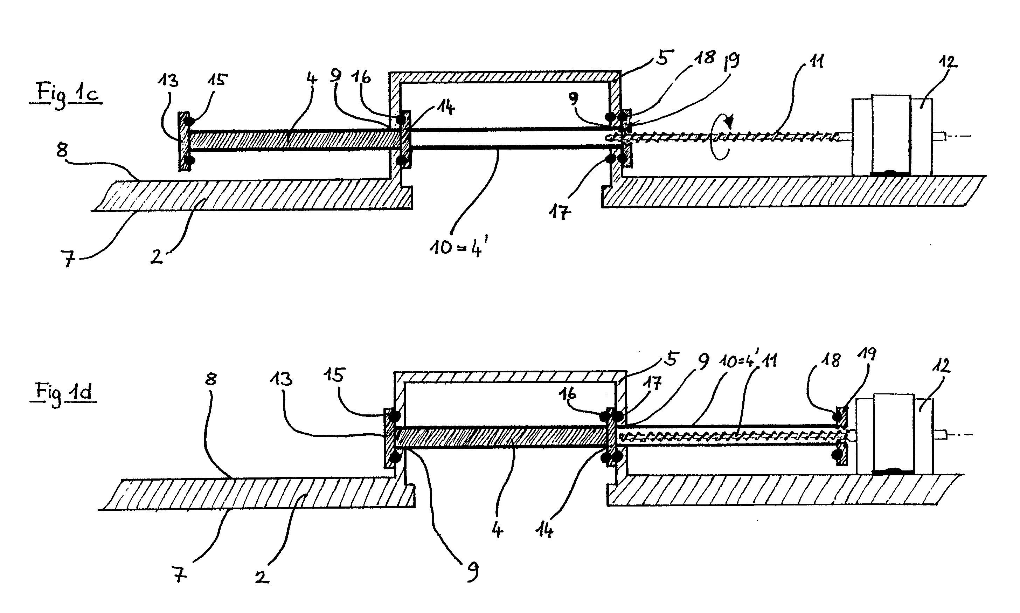

Fig. 1b
einen Querschnitt durch die Tür und den Griff längs der Linie A-B der Fig. 1a;

Fig. 1c und 1d
weitere Querschnitte längs der Linie C-D durch die Tür und den Griff der Desinfektionsspülmaschine gemäß Fig. 1a in zwei verschiedenen Grenzstellungen des Türgriffes;

Fig. 2a und 2c
einen Querschnitt durch Tür und Türgriff einer Desinfektionsspülmaschine nach einem zweiten Ausführungsbeispiel der Erfindung in zwei verschiedenen Grenzstellungen;

Fig. 2b und 2d
perspektivische Darstellungen der Außenseite der Tür in den beiden Grenzstellungen gemäß Fig. 2a und 2c;

Fig. 3a
einen Querschnitt einer Tür und eines Türgriffes einer Desinfektionsspülmaschine nach einem dritten Ausführungsbeispiel der Erfindung mit einem ringförmigen, gegenüber der Tür beweglichen Türgriff;

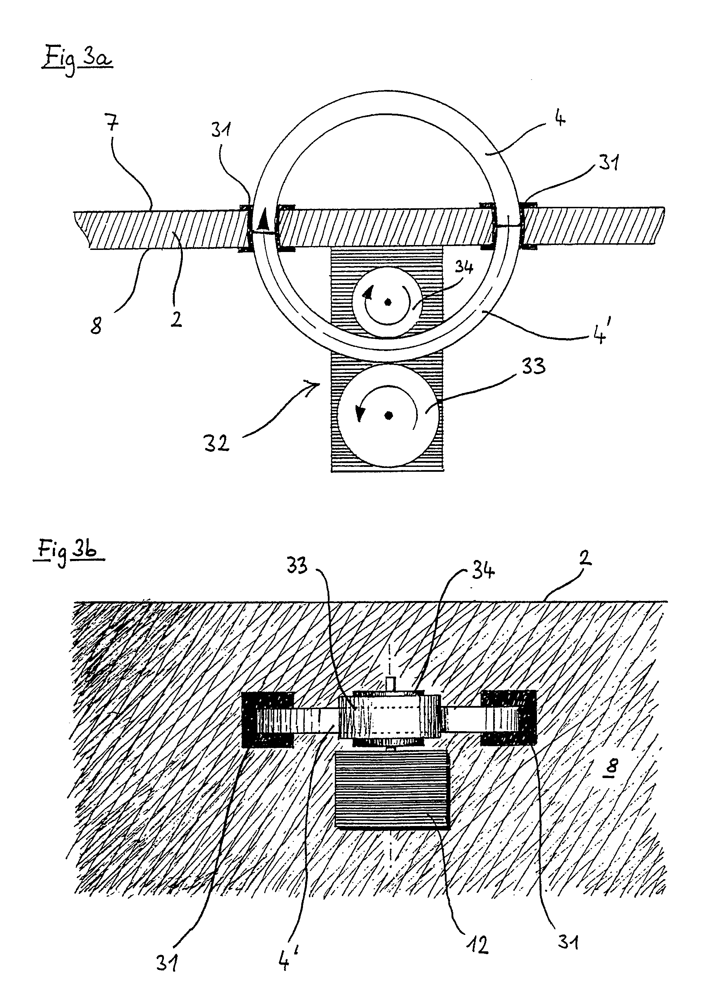

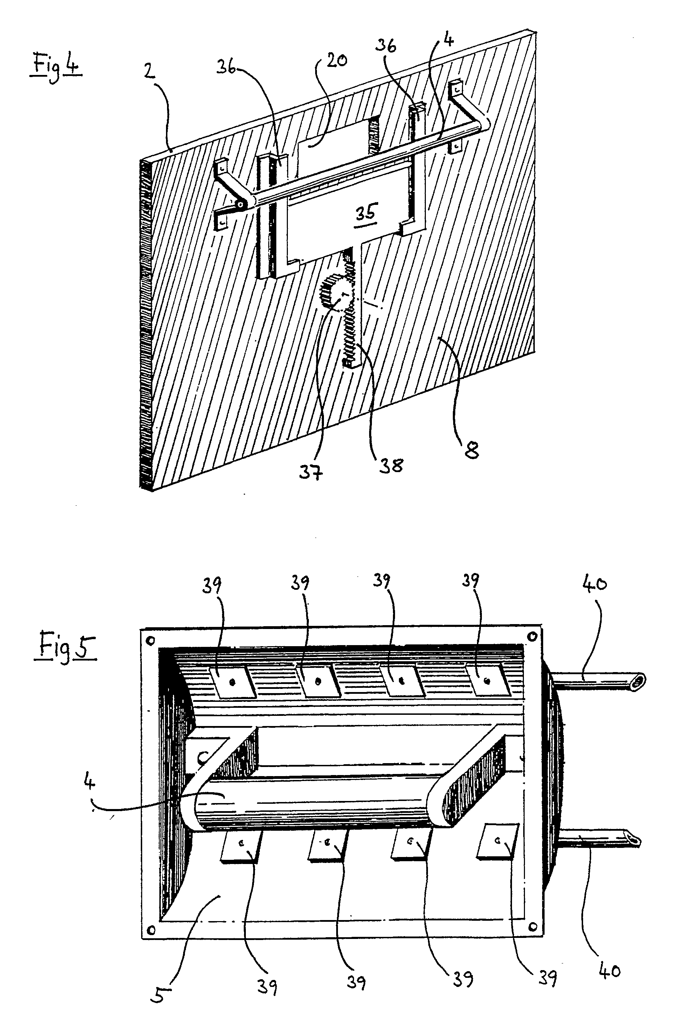

Fig. 3b
eine Ansicht der Innenseite der Tür des Ausführungsbeispieles der Fig. 3a;

Fig. 4
eine perspektivische Ansicht auf die Innenseite der Tür einer Desinfektionsspülmaschine gemäß einem vierten Ausführungsbeispiel der Erfindung mit einer verschiebbaren Klappe, die ein Durchgriffenster zum Erreichen des Türgriffes öffnet bzw. schließt;

Fig. 5
einen Türgriff mit Griffmulde und Sprühdüsen einer Desinfektionsspülmaschine gemäß einem fünften Ausführungsbeispiel der Erfindung;

Fig. 6
ein Ausschnitt der Tür und des Türgriffes einer Desinfektionsspülmaschine gemäß einem sechsten Ausführungsbeispiel der Erfindung;

Fig. 7
einen Türgriff mit Griffmulde einer Desinfektionsspülmaschine gemäß einem siebten Ausführungsbeispiel der Erfindung;

Fig. 8
eine schematische, perspektivische Ansicht der Vorderseite einer Desinfektionsspülmaschine mit einer auf Umschlag gelagerten Tür gemäß einem achten Ausführungsbeispiel der Erfindung;

und

Fig. 9

eine Ansicht eines Türgriffes mit Griffmulde und einer Induktionsspule zum Erwärmen und damit Desinfizieren des Türgriffes gemäß einem neunten Ausführungsbeispiel der Erfindung.

Fig. 1a zeigt schematisch eine Desinfektionsspülmaschine 1 mit einer Tür 2, die am Gehäuse der Maschine über ein Scharnier 3 befestigt ist. Ein Türgriff 4 ist hier als Griffrohr ausgebildet, das sich längs durch eine in der Tür 2 versenkte Griffmulde 5 erstreckt. Mit 6 sind schematisch verschiedene Bedienelemente der Spülmaschine 1 angedeutet.

Im Querschnitt der Fig. 1b ist die Anordnung der Griffmulde 5 und des Türgriffes 4 besser zu erkennen. Die Griffmulde 5 ist zur Türaußenseite 7 offen, so daß man von außen den Türgriff 4 ergreifen kann. Die Griffmulde erstreckt sich daher in Richtung zur Türinnenseite 8.

Wie aus den Fig. 1c und 1d zu erkennen ist, hat die Griffmulde zwei seitliche, miteinander fluchtende Bohrungen 9, deren Achse parallel zur Ebene der Tür 2 liegt. Der Türgriff 4 läßt sich in Richtung seiner Längsachse in diesen Bohrungen 9 verschieben, so daß er in seiner einen Grenzstellung (Fig. 1d) im Inneren der von außen zugänglichen Griffmulde 5 und in seiner anderen Grenzstellung (Fig. 1c) außerhalb der Griffmulde 5 und damit im Inneren des Waschraumes der Desinfektionsspülmaschine liegt. In der letzt genannten Grenzstellung wird er zusammen mit dem Waschgut gewaschen und desinfiziert.

Im einzelnen ist an einem Ende des Türgriffes 4 eine rohrförmige Griffverlängerung 10 mit Innengewinde angebracht, in das eine Spindel 11, die von einem Elektromotor 12 angetrieben wird, eingreift. Dreht der Motor 12 die Spindel 11, so läßt sich der Türgriff 4 zwischen den beiden dargestellten Grenzstellungen verschieben.

Die Griffverlängerung 10 kann auch als Teil des gesamten Türgriffes verwendet werden (Teil 4 ), wobei alternierend entweder der Teil 4 oder der Teil 4 in der Griffmulde ist und der andere Teil im Inneren des Waschraumes. Die Steuerung kann dann so ausgebildet sein, daß nach jedem Waschvorgang das jeweils zuletzt gewaschene Teil in die Griffmulde gefahren wird.

Die Bohrungen 9 können auf verschiedene Weise gegenüber dem Waschraum der Spülmaschine abgedichtet sein. Bei einer nicht dargestellten Variante können in den Bohrungen 9 Dichtungsringe vorgesehen sein. Bei der dargestellten Variante hat der Türgriff 4 zwei Endflansche 13 und 14, an denen Dichtungsringe 15 bzw. 16 angebracht sind. Der eine Endflansch 14 liegt dabei innerhalb der Griffmulde 5, während der andere Endflansch 13 ständig im Waschraum liegt. An dem freien Ende der Griffverlängerung 10 ist ein weiterer Endflansch 19 mit zur Außenseite der Griffmulde 5 hinweisenden Dichtungsring 18 vorgesehen. Selbstverständlich sind alle bekannten Dichtungsarten hier möglich, wobei darauf hinzuweisen ist, daß bei Desinfektionsspülmaschinen Dichtungen ausreichen, die spritzwasserfest sind, da der Waschraum von üblichen Desinfektionsspülmaschinen nicht unter Überdruck gegenüber der Außenseite gerät.

Der Motor 12 wird vom (nicht dargestellten) Programmsteuerwerk der Desinfektionsspülmaschine gesteuert, beispielsweise in der Weise, daß der Türgriff 4 bei Beginn des Waschvorganges in den Waschraum gefahren wird (Fig. 1c) und bei Beendigung des Wasch- und ggf. auch Trocknungsvorganges wieder zurück in die Griffmulde 5 gefahren wird. Damit ist sichergestellt, daß der Türgriff automatisch bei jedem Waschvorgang zwangsweise desinfiziert wird.

Im dargestellten Ausführungsbeispiel (vgl. insbesondere Fig. 1b) hat der Türgriff 4 einen rechteckigen Querschnitt, womit sichergestellt ist, daß sich der Türgriff 4 nicht um seine eigene Achse drehen kann und dann ein Verschieben nicht mehr möglich ist.

Nach einer anderen, hier nicht dargestellten Variante könnte statt des Motors 12 und der Spindel 11 auch ein Elektromagnet vorgesehen sein, dessen Anker durch die Griffverlängerung 10 gebildet ist. Ebenso ist es möglich, statt der Spindel 11 ein Zahnrad zu verwenden, das in Zähne an der Außenseite der Griffverlängerung eingreift. Die Griffverlängerung wäre dann also als Zahnstange ausgebildet.

Eine weitere, nicht dargestellte Variante dieses Ausführungsbeispieles besteht darin, daß der Türgriff 4 über Gestänge, Seilzüge oder ähnliches durch das Schließen der Tür in die in Fig. 1c dargestellte Lage bewegt wird, wobei gleichzeitig eine Feder gespannt wird, die in der in Fig. 1c dargestellten Endlage jedoch verriegelt wird. Am Ende des Waschvorganges kann diese Verriegelung von der Steuerung durch einen Elektromagneten gelöst werden, so daß dann die Feder den Türgriff dann wieder in die in Fig. 1d dargestellte andere Endlage zurückbewegt.

Fig. 2a bis 2d zeigt ein zweites Ausführungsbeispiel der Erfindung, bei dem der Türgriff feststeht und die Griffmulde durch eine bewegliche Klappe gebildet wird, die den Griff in der einen Grenzstellung von außen und in der anderen Grenzstellung vom Waschraum her zugänglich läßt.

Im einzelnen hat die Tür 2 im Bereich des Griffs 4 ein Fenster 20, in das eine halbzylindrische Klappe 21 mit Seitenwänden 22 eingesetzt ist, wobei das Innere der Klappe gleichzeitig die Griffmulde bildet. Diese Klappe ist zwischen zwei Grenzstellungen verschwenkbar. In den Darstellungen der Fig. 2a und 2b ist diese Klappe 21 in der Grenzstellung, bei der der Türgriff 4 von der Türaußenseite 7 her zugänglich ist. In der anderen Grenzstellung der Fig. 2c und 2d verschließt die Klappe 21 dagegen das Fenster 20 zur Außenseite 7 hin, so daß der Türgriff 4 zum Waschraum hin bloßliegt und beim Desinfektionsvorgang ebenfalls desinfiziert wird.

Zur Abdichtung und als Anschlag für zumindest die eine Grenzstellung (Fig. 2c) besitzt die halbzylindrische Klappe 21 radial nach außen vorstehende, achsparallele Leisten 23 und 24, die sich über die volle Länge des Zylinders erstrecken und die mit in der Tür 4 angebrachten achsparallelen Dichtungen 25 bzw. 26 in Anschlag kommen, wenn die Klappe in der in Fig. 2c und 2d dargestellten Grenzstellung ist. Die stirnseitigen Seitenwände 22 weisen in der Ebene der Leisten 23 und 24 im wesentlichen radial verlaufende Leisten 27 bzw. 28 auf, an denen Dichtungsstreifen angebracht sind. Um eine einwandfreie Anlage der Leisten 23 und 24 an der Tür zu gewährleisten, ist die Tür im Anschlagbereich dieser Leisten mit Verjüngungen 29 versehen, deren Oberfläche auf die Drehachse ausgerichtet ist, so daß die Leisten dort möglichst plan anliegen.

Wie in den Fig. 2b und 2d angedeutet, wird die Klappe 21 durch einen Motor 12 und eine Welle 30 angetrieben. Auch hier wird der Motor vom Steuerwerk der Desinfektionsspülmaschine gesteuert, so daß sichergestellt ist, daß bei jedem Spülvorgang die Klappe in der in Fig. 2c und 2d dargestellten Grenzstellung ist, so daß der Griff desinfiziert wird. Nach Beendigung des Spülvorganges wird dann die Klappe wieder "geöffnet", so daß der Türgriff 4 von außen zugänglich ist.

Statt der im Ausführungsbeispiel der Fig. 2a bis 2d gezeigten Dichtungen können auch sonstige bekannte Dichtungen verwendet werden, beispielsweise rings um das viereckige Fenster 20 herum laufende Gummilippen, die eine ausreichende Dichtung gegen Spritzwasser sicherstellen. Statt eines Motors 30 kann auch hier ein Elektromagnet verwendet werden, der über ein Gestänge die Klappe betätigt. Schließlich kann auch die im Zusammenhang mit Fig. 1 erläuterte mechanische Betätigung der Klappe über das Schließen der Tür vorgesehen sein.

Fig. 3a und 3b zeigt eine dritte Variante der Erfindung, bei der der Türgriff 4 als geschlossener Ring ausgebildet ist, der so in zwei abgedichtete (Dichtungen 31) Durchgangsöffnungen in der Tür 2 eingesetzt ist, daß sich ein Teil 4 an der Außenseite 7 und ein anderer Teil 4 an der Innenseite 8 der Tür 2 befindet. Ein Antriebsmechanismus 32, der hier aus einem Elektromotor 12 mit Antriebsrolle 33 und einer Andruckrolle 34 besteht, dreht den ringförmigen Türgriff in Abhängigkeit des Steuerwerkes der Desinfektionsspülmaschine, so daß der vor dem Spülvorgang an der Außenseite 7 liegende Abschnitt des Türgriffes 4 sich beim Waschvorgang im Spülraum befindet, dort desinfiziert und nach Beendigung des Waschvorganges wieder zur Außenseite bewegt wird. Um einen guten Transport zu gewährleisten, ist der Türgriff 4 als Ring mit rechteckigem Querschnitt ausgebildet, so daß die Antriebsrolle 33 und die Andruckrolle 34 ausreichend Reibfläche vorfinden.

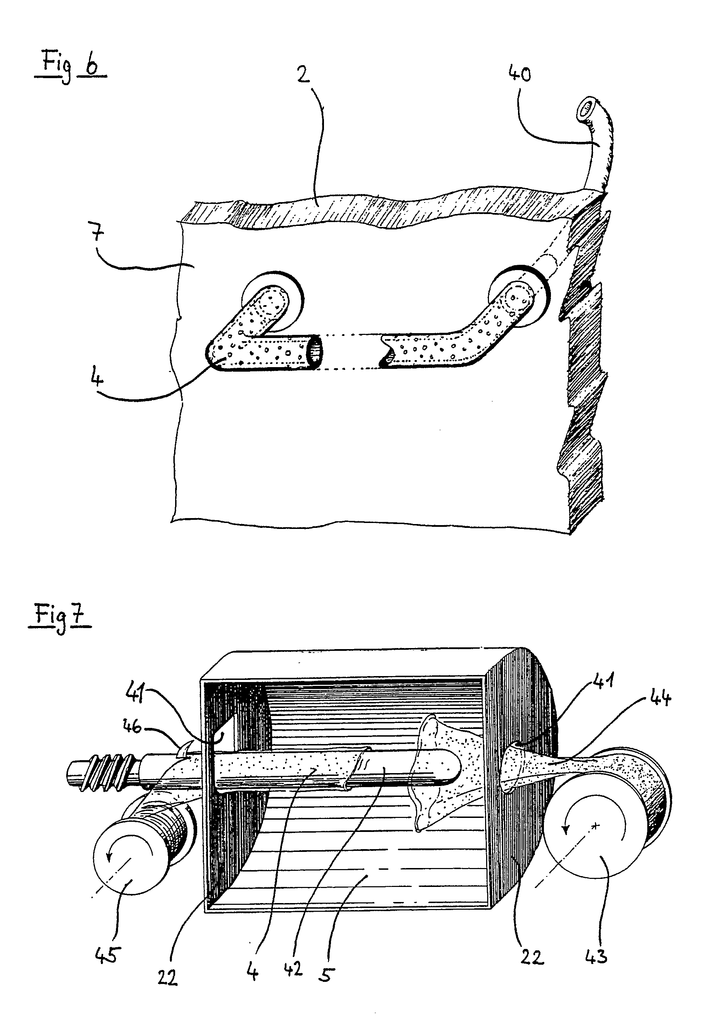

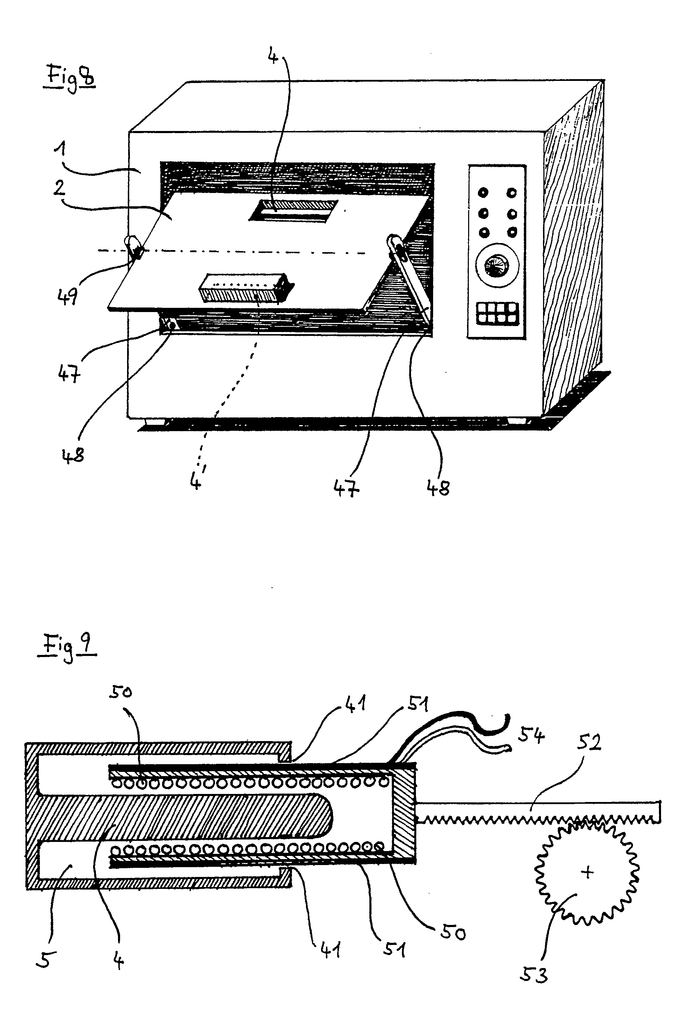

Fig. 4 zeigt ein viertes Ausführungsbeispiel der Erfindung, bei dem der Türgriff 4 fest an der Innenseite 8 der Tür 2 angebracht ist und durch ein Fenster 20 in der Tür von außen her zugänglich ist. Dieses Fenster wird durch eine verschiebbare Klappe 35 geöffnet bzw. verschlossen. Im dargestellten Ausführungsbeispiel ist diese Klappe in einem an der Türinnenseite 8 angebrachten Führungsprofil 36 geführt und wird durch einen nicht dargestellten Elektromotor, ein Ritzel 37 und eine mit der Klappe 35 verbundene und mit dem Ritzel 37 in Eingriff stehende Zahnstange verschoben. Der Motor wird auch hier durch das Steuerwerk der Desinfektionsspülmaschine so gesteuert, daß während des Waschvorganges das Fenster 20 verschlossen ist. Selbstverständlich befinden sich zwischen der Klappe 35 und der Umrandung des Fensters 20 Dichtungen. Auch hier können Modifikationen des Antriebes der Klappe 35 analog zu den oben erläuterten Ausführungsbeispielen vorgesehen sein.

Fig. 5 zeigt ein fünftes Ausführungsbeispiel der Erfindung, bei dem der Türgriff 4 in der Griffmulde 5 angeordnet ist und in der Griffmulde mehrere auf den Türgriff 4 gerichtete Sprühdüsen 39 vorgesehen sind, die über ein oder mehrere Schläuche bzw. Rohre 40 mit einer Quelle für Desinfektionsflüssigkeit verbunden sind. Eine nicht dargestellte Fördereinrichtung für die Desinfektionsflüssigkeit, beispielsweise eine Förderpumpe, eine Druckmittelquelle in Verbindung mit einem Magnetventil oder ähnliches sorgen unter Steuerung des Steuerwerkes dafür, daß der Türgriff 4 zu vorgegebenen Zeitpunkten, beispielsweise jedesmal bei Beendigung eines Waschvorganges mit Desinfektionslösung besprüht wird.

Fig. 6 zeigt eines sechstes Ausführungsbeispiel der Erfindung, bei dem der Türgriff 4 aus porösem Material, wie z.B. Sintermetall, besteht und über einen Schlauch bzw. ein Rohr 40 ebenfalls mit einer Quelle für Desinfektionslösung angeschlossen ist. Der Türgriff wird dann unter Steuerung des Steuerwerkes der Spülmaschine mit Desinfektionslösung von innen her getränkt und speichert in seinen Poren diese Lösung. Im dargestellten Ausführungsbeispiel ist der Türgriff 4 als innen hohles Rohr aus porösem Material ausgebildet, womit eine möglichst gleichmäßige Durchtränkung des Griffs mit Desinfektionslösung sichergestellt ist. Zweckmäßig ist es auch hier, wenn der Griff während vorbestimmter Phasen des Spülvorganges mit neuer Desinfektionslösung beaufschlagt wird.

Fig. 7 zeigt ein siebtes Ausführungsbeispiel der Erfindung, bei der der Türgriff dadurch steril ist, daß seine Oberfläche aus einer sich erneuernden, sterilen Folie besteht. Auch hier befindet sich der Türgriff in einer Griffmulde 5, die in ihren Seitenwänden 22 miteinander fluchtende Öffnungen 41 aufweist, durch die ein Trägerelement 42 des Griffs 4 hindurchragt. Neben der Griffmulde ist eine Vorratsrolle 43 für eine Schlauchfolie 44 vorgesehen, die über das Trägerelement 42 gezogen wird und zusammen mit dem Trägerelement den Griff 4 bildet. Auf der anderen Seite der Griffmulde ist eine Entsorgungsrolle 45 vorgesehen, auf die verbrauchte Abschnitte der Schlauchfolie 41 aufgewickelt werden. Zum Lösen der Schlauchfolie 41 von dem Trägerelement 42 ist am entsorgungsseitigen Ende des Trägerelementes ein radial abstehendes Messer 46 vorgesehen, das die Schlauchfolie aufschlitzt. Die Entsorgungsrolle 45 wird durch einen nicht dargestellten Motor in von dem Steuerwerk der Spülmaschine vorgegebenen Intervallen in Pfeilrichtung angetrieben, so daß die im Bereich der Griffmulde befindlichen Abschnitte der Schlauchfolie 41 in diesen Intervallen erneuert werden. Zum Aufziehen einer neuen Schlauchfolie ist das Trägerelement 42 in seiner Längsrichtung verschiebbar, so daß das freie Ende des Trägerelementes 42 frei zugänglich ist und eine neue Schlauchfolie aufgezogen werden kann. Das Steuerwerk treibt den Motor für die Drehung der Entsorgungsrolle 45 vorzugsweise immer dann an, wenn eine Spülvorgang abgeschlossen ist, so daß für die darauf folgende Entnahme stets frische Schlauchfolie im Bereich des Griffs vorhanden ist.

Fig. 8 zeigt ein weiteres Ausführungsbeispiel der Erfindung, bei dem die Tür beidseitig je einen Türgriff 4 aufweist und die Tür zwei auf "Umschlag" in Doppelscharnieren gehalten ist, so daß abwechselnd beide Seiten und damit beide Türgriffe zum Spülraum weisen. Im dargestellten Ausführungsbeispiel ist die Tür 2 an zwei Scharnierleisten 47 drehbar gelagert, wobei das eine Ende der Scharnierleisten 47 in Schwenklagern 48 am Gehäuse der Desinfektionsspülmaschine 1 befestigt ist, während das andere Ende der Scharnierleisten 47 ein Drehlager 49 für das Drehen der Tür 2 enthält. Dieses Drehlager 49 ist vorzugsweise so ausgebildet, daß es die Drehung der Tür 2 nur in einer Drehrichtung erlaubt, was beispielsweise durch Rastklinken erreicht wird. Damit ist sichergestellt, daß nach jedem Öffnen der Tür ein Schließen nur mit um 180° gekippter Tür möglich ist, womit zwangsweise jeder der Türgriffe 4 und 4 abwechselnd bei jedem zweiten Spülvorgang desinfiziert wird.

Fig. 9 zeigt ein neuntes Ausführungsbeispiel der Erfindung, bei dem der Türgriff 4 thermisch sterilisiert wird. Nach diesem Ausführungsbeispiel ist der Türgriff 4 einseitig an der Griffmulde 5 befestigt und ragt mit seinem freien Ende durch die Öffnung 41 aus der Griffmulde heraus. Zur thermischen Desinfektion kann eine Induktionsspule 50 temporär über den Türgriff 4 gefahren werden und kann dessen Oberfläche auf eine solche Temperatur erhitzen, daß alle dort befindlichen Keime abgetötet sind. Speist man die Induktionsspule 50 mit hochfrequentem Strom, so wird durch den Skineffekt nur die oberste Grenzschicht des Türgriffes 4 erhitzt, was für den Zweck der Sterilisierung bzw. Desinfektion ausreicht. Damit wird auch erreicht, daß sich der Türgriff sehr schnell wieder abkühlt und wieder benutzbar ist. Zur Sicherung gegen Verbrennen hat die Induktionsspule 50 einen thermisch isolierenden Mantel 51. Unter Steuerung durch das Steuerwerk der Desinfektionsspülmaschine wird die Induktionsspule 50 zu vorbestimmten Zeitpunkten des Waschvorganges, vorzugsweise gegen Ende des Waschvorganges in ihre Wirkposition gefahren. Hierzu ist die Induktionsspule 50 mit einer Zahnstange 52 verbunden, die durch ein Ritzel 53 von einem Motor angetrieben wird. Flexible Anschlußdrähte 54 sind mit einem Hochfrequenzgenerator verbunden. Zur Sicherung gegen Verbrennungen ist zusätzlich vorgesehen, daß die Induktionsspule 50 nach Abschalten des Erregerstromes noch solange in ihrer Wirkstellung bleibt, bis der Türgriff 4 auf eine Temperatur abgekühlt ist, die mit Sicherheit keine Verbrennungen verursachen kann.

Obwohl die Ausführungsbeispiele der Erfindung im Zusammenhang mit einer Desinfektionsspülmaschine erläutert wurden, sei darauf hingewiesen, daß zumindest einige der Ausführungsbeispiele auch zur Desinfektion sonstiger Türgriffe, wie z.B. Griffe von Schränken für sterile Lagerung von Operationsbesteck, Türen von Operationssälen, Zimmern in Krankenhäusern etc., eingesetzt werden kann.

In bestimmten Fällen, beispielsweise bei Zimmertüren oder ähnlichem ist nach der Erfindung vorgesehen, die Desinfektionseinrichtung in vorgegebenen Zeitintervallen wirksam zu schalten, beispielsweise durch eine Zeitschaltuhr, Taktsteuerung oder ähnliches. Nach einer weiteren Variante der Erfindung kann die Desinfektionseinrichtung in Abhängigkeit von der jeweiligen Betätigung des Türgriffes wirksam geschaltet werden, beispielsweise nach jeder einzelnen Betätigung oder auch nach einer vorgegebenen Anzahl von Betätigungen, wobei im letzteren Fall ein Zähler vorgesehen ist, der bei Erreichen eines vorgegebenen Zählerstandes den Desinfektionsvorgang einleitet.

Ein weiterer Vorteil der Ausführungsbeispiele der Fig. 2, 5 und 8 liegt darin, daß auch die dort vorhandene und ebenfalls keimrelevante Griffmulde bzw. im Ausführungsbeispiel der Fig. 8 sogar die gesamte Türaußenseite desinfiziert wird.

**Patentansprüche**

1. Desinfektionseinrichtung für Türgriffe (4), insbesondere für Türgriffe an Desinfektionsspülmaschinen (1), gekennzeichnet durch Mittel (5, 10, 11, 12; 20, 21, 30; 31, 32; 35, 37, 38; 39; 40; 44; 47, 48, 49; 50), die in Abhängigkeit von einer übergeordneten Funktion zwangsweise eine automatische Desinfektion des Türgriffes (4, 4 ) gewährleistet.

2. Desinfektionseinrichtung für Türgriffe an Desinfektionsspülmaschinen nach Anspruch 1, dadurch gekennzeichnet, daß ein zu desinfizierender Teil des Türgriffs (4, 4 ) während des Spülvorganges im Waschraum der Spülmaschine (1) angeordnet ist.

3. Desinfektionseinrichtung nach Anspruch 2, gekennzeichnet durch Transporteinrichtungen (10, 11, 12; 32; 49), die den Türgriff (4, 4 ) in den Waschraum verfahren.

4. Desinfektionseinrichtung nach Anspruch 2, dadurch gekennzeichnet, daß eine Klappe (21; 35) vorgesehen ist, die in ihrer einen Grenzstellung den feststehenden Türgriff (4) von der Außenseite (7) der Spülmaschine (1) und in der anderen Grenzstellung nur vom Waschraum her zugänglich läßt.

5. Desinfektionseinrichtung nach Anspruch 1, dadurch gekennzeichnet, daß auf den Türgriff (4) gerichtete Sprühdüsen (39) für Desinfektionsflüssigkeit vorgesehen sind und daß diese Sprühdüsen (39) an eine gesteuerte Fördereinrichtung angeschlossen sind.

6. Desinfektionseinrichtung nach Anspruch 1, dadurch gekennzeichnet, daß der Türgriff (4) aus flüssigkeitsdurchlässigem Material besteht und an eine gesteuerte Einrichtung für Desinfektionsflüssigkeit angeschlossen ist.

7. Desinfektionseinrichtung nach Anspruch 1, dadurch gekennzeichnet, daß der Türgriff (4) durch ein Trägerelement (42) und einen dieses Trägerelement umhüllenden sterilen Folienschlauch (44) gebildet ist und daß eine gesteuerte Transporteinrichtung (45) zur Ver- und Entsorgung von Abschnitten des Folienschlauches (44) vorgesehen ist.

8. Desinfektionseinrichtung nach Anspruch 1, dadurch gekennzeichnet, daß der Türgriff (4) temporär im Bereich des Magnetfeldes einer Induktionsspule (50) angeordnet ist.

9. Desinfektionseinrichtung nach Anspruch 2 oder 3, dadurch gekennzeichnet, daß der Türgriff (4, 4 ) derart verschieblich in einer Griffmulde (5) der Tür (2) angeordnet ist, daß sich eine Hälfte (4 oder 4 ) im Inneren des Waschraumes und die andere Hälfte (4 oder 4) in der Griffmulde befindet und daß eine Transporteinrichtung (10, 11, 12) vorgesehen ist, die den Griff zwischen zwei Grenzstellungen verfährt.

10. Desinfektionseinrichtung nach Anspruch 4, dadurch gekennzeichnet, daß in der Tür (2) ein Fenster (20) vorgesehen ist, in dem der Türgriff (4) fest angeordnet ist und daß eine um die Zylinderachse schwenkbare, halbzylindrische Klappe (21) mit Seitenwänden (22) vorgesehen ist, die das Fenster (20) verschließt.

11. Desinfektionseinrichtung nach Anspruch 3, dadurch gekennzeichnet, daß der Türgriff ein geschlossener Ring ist, der so in die Tür (2) eingesetzt ist, daß sich seine eine Hälfte (4 oder 4 ) diesseits der Türaußenseite (7) und seine andere Hälfte (4 oder 4) jenseits der Türaußenseite (7) befindet und daß eine Transporteinrichtung (32) vorgesehen ist, die den Ring so dreht, daß die Griffhälften (4, 4 ) ihre Position wechseln.

12. Desinfektionseinrichtung nach Anspruch 4, dadurch gekennzeichnet, daß der Türgriff (4) fest an der Türinnenseite (8) angebracht und durch ein Fenster (20) in der Tür von außen zugänglich ist und daß dieses Fenster (20) durch eine Klappe (35) mittels einer Transporteinrichtung (37, 38) verschließbar ist.

13. Desinfektionseinrichtung nach Anspruch 6, dadurch gekennzeichnet, daß der Türgriff (4) aus porösem, flüssigkeitsspeicherndem Material ist, innen hohl ist und erlaubt, daß Flüssigkeit von innen zur Oberfläche des Türgriffes gelangt.

14. Desinfektionseinrichtung nach Anspruch 7, dadurch gekennzeichnet, daß der Folienschlauch (44) von einer auf der einen Seite der Griffmulde (5) angeordneten Vorratsrolle (43) zu einer auf der anderen Seite der Griffmulde (5) angeordneten Entsorgungsrolle (45) transportierbar ist und daß am entsorgungsseitigen Ende des Trägerelementes (42) ein Messer (46) angeordnet ist, das den Folienschlauch (44) in Längsrichtung auftrennt.

15. Desinfektionseinrichtung nach Anspruch 3, dadurch gekennzeichnet, daß auf der Außen- und der Innenseite (7 und 8) der Tür (2) je ein Türgriff (4, 4 ) angeordnet ist, daß die Tür so in Doppelgelenken (48, 49) gelagert ist, daß ihre beiden Seiten (7, 8) vertauschbar sind und daß das eine der Gelenke (49) so ausgebildet ist, daß es eine Drehung nur in einer Drehrichtung zuläßt.

16. Desinfektionseinrichtung nach Anspruch 8, dadurch gekennzeichnet, daß die Induktionsspule (50) durch eine Transporteinrichtung (52, 53) so verfahrbar ist, daß sie in ihrer einen Grenzstellung den Türgriff (4) übergreift und in ihrer anderen Grenzstellung frei gibt.

Fig 1a

Fig 1b

Fig 1c

Fig 1d

EP 0 448 122 A2

Fig 2a

Fig 2c

Fig 2b

Fig 3d

10

_Fig 3a_

_Fig 3b_

Fig 4

Fig 5

<u>Fig 6</u>

<u>Fig 7</u>

Fig 8

Fig 9